# EUROPEAN PATENT SPECIFICATION

(11) **EP 0 901 500 B1**
(45) Date of publication and mention of the grant of the patent: **19.11.2003**
(21) Application number: 97921334.5
(22) Date of filing: 28.04.1997
(51) Int. Cl.: C07H 21/00, C07F 9/24, C07F 9/48, C07H 19/10, C07H 19/20

(54) **IN SITU PREPARATION OF NUCLEOSIDE PHOSPHORAMIDITES AND THEIR USE IN SYNTHESIS OF OLIGONUCLEOTIDES**
IN SITU HERSTELLUNG VON NUKLEOSID-PHOSPHORAMIDITEN UND IHRE VERWENDUNG IN DER OLIGONUKLEOTIDSYNTHESE
PREPARATION IN SITU DE PHOSPHORAMIDITES DE NUCLEOSIDES ET LEUR UTILISATION POUR LA SYNTHESE D'OLIGONUCLEOTIDES

(30) Priority: 03.05.1996 US 642653
(43) Date of publication of application: 17.03.1999
(73) Proprietor: Avecia Biotechnology Inc, Milford, MA 10757 (US)
(72) Inventor: ZHANG, Zhaoda, Shrewsbury, MA 01545 (US); TANG, Jin-Yan, Schrewsbury, MA 01545 (US)
(74) Representative: Revell, Christopher
(86) International application number: US9706777
(87) International publication number: WO97042208

(56) References cited:
- WO-A-84/04749
- WO-A-86/07362
- DE-A- 1 917 884
- DE-A- 3 332 068
- US-A- 3 320 251
- DATABASE WPI Derwent Publications Ltd., London, GB; AN 87-302875 XP002035768 & JP 62 212 394 A (NIPPON ZEON KK)
- TETRAHEDR. LETT., vol. 33, 1992, pages 2357-60, XP002035761 J.H. VAN BOOM ET AL.: "Synthesis of Alkylphosphon(othio)ate Analogues of DNA"
- TETRAHEDR. LETT., vol. 25, 1984, pages 375-8, XP002035762 S.L. BEAUCAGE: "A Simple and Efficient Preparation of Deoxynucleoside Phosphoramidites in situ"
- TETRAHEDRON, vol. 50, 1994, pages 5719-34, XP002035763 W. ENGELS ET AL.: "New Prolin derived Chiral Building Blocks for Nucleoside Methylphosphonate Synthesis"
- INORG. CHEM., vol. 29, 1990, pages 2608-13, XP002035764 A.D. NORMAN ET AL.: "Synthesis of Mono- and Diphosphorus Phosphazane Oligomer/ Polymer Precursors"
- Z. ANORG. ALLG. CHEM., vol. 588, 1990, pages 147-66, XP002035765 E. FLUCK ET AL.: "Bemerkungen zur Synthese von Difluorphosphoranen"
- SYN. INORG. METAL-ORG. CHEM., vol. 2, 1972, pages 13-7, XP002035766 S. CHAN ET AL.: "Lithium Dimethylamide in the Preparation of Phosphorous- and Silicon-Nitrogen Compounds"
- J. ORGANOMET. CHEM., vol. 378, no. 1, 1989, pages 17-31, XP002035767 W. KUCHEN ET AL.: "Reaktionen koordinierter Liganden"

## Description

### BACKGROUND OF THE INVENTION

### Field of the Invention

The invention relates to the chemical synthesis of oligonucleotides and to chemical entities useful in such synthesis.

### Summary of the Related Art

Oligonucleotides have become indispensable tools in modern molecular biology, being used in a wide variety of techniques, ranging from diagnostic probing methods to PCR to antisense inhibition of gene expression. This widespread use of oligonucleotides has led to an increasing demand for rapid, inexpensive and efficient methods for synthesizing oligonucleotides.

The synthesis of oligonucleotides for antisense and diagnostic applications can now be routinely accomplished. See *e.g*., Methods in Molecular Biology, Vol 20: Protocols for Oligonucleotides and Analogs pp. 165-189 (S. Agrawal, Ed., Humana Press, 1993); Oligonucleotides and Analogues: A Practical Approach, pp. 87-108 (F. Eckstein, Ed., 1991); and Uhlmann and Peyman, supra. Agrawal and Iyer, Curr. Op. in Biotech. 6: 12 (1995); and Antisense Research and Applications (Crooke and Lebleu, Eds., CRC Press, Boca Raton, 1993). Early synthetic approaches included phosphodiester and phosphotriester chemistries. Khorana et al., J. Molec. Biol. 72: 209 (1972) discloses phosphodiester chemistry for oligonucleotide synthesis. Reese, Tetrahedron Lett. 34: 3143-3179 (1978), discloses phosphotriester chemistry for synthesis of oligonucleotides and polynucleotides. These early approaches have largely given way to the more efficient phosphoramidite and H-phosphonate approaches to synthesis. Of these, the phosphoramidite approach has become the most popular for most applications. Beaucage and Caruthers, Tetrahedron Lett. 22: 1859-1862 (1981), discloses the use of deoxynucleoside phosphoramidites in polynucleotide synthesis. The phosphoramidite approach has been used to synthesize oligonucleotides having a variety of modified internucleoside linkages. Agrawal and Goodchild, Tetrahedron Lett. 28: 3539-3542 (1987), teaches synthesis of oligonucleotide methylphosphonates using phosphoramidite chemistry. Connolly et al., Biochemistry 23: 3443 (1984), discloses synthesis of oligonucleotide phosphorothioates using phosphoramidite chemistry. Jager el al., Biochemistry 27: 7237 (1988), discloses synthesis of oligonucleotide phosphoramidates using phosphoramidite chemistry. Solid phase synthesis of oligonucleotides by the phosphoramidite approach can be varied for different applications, but ordinarily involves the same generalized protocol. Briefly, this approach comprises anchoring the 3'-most nucleoside to a solid support functionalized with amino and/or hydroxyl moieties and subsequently adding the additional nucleosides in stepwise fashion. Desired internucleoside linkages are formed between the 3' phosphoramidite group of the incoming nucleoside and the 5' hydroxyl group of the 5'-most nucleoside of the nascent, support-bound oligonucleotide.

Refinement of methodologies is still required, however, particularly when making a transition to large-scale synthesis (10umol to 1 mmol and higher). See Padmapriya et al., Antisense Res. Dev. 4: 185 (1994). Several modifications of the standard phosphoramidite methods have already been reported to facilitate the synthesis and isolation of oligonucleotides. See *e.g*., Padmapriya et al., *supra*; Ravikumar et al., Tetrahedron 50: 9255 (1994); Theisen et al., Nucleosides & Nucleotides 12: 43 (1994); and Iyer et al., Nucleosides & Nucleotides 14: 1349 (1995) (Kuijpers et al., Nucl. Acids Res. 18: 5197 (1990); and Reddy et al., Tetrahedron Lett. 35: 4311 (1994).

A major limiting factor for cost efficient synthesis of oligonucleotides is the time and cost required to make and purify the monomeric nucleoside phosphoramidites. Bodepudi *et al*., Chem. Res. Toxicol. 5: 608-617, discloses that the preparation of phosphoramidites from 2'-deoxy-7,8-dihydro-8-oxoguanosine and 2'-deoxy-7,8-dihydro-8-oxoadenosine according to the standard procedure results in extensive decomposition of the phosphoramidites during purification due to their instability and sensitivity to water. One potential approach to overcome these problems is to generate the phosphoramidite *in situ* as the oligonucleotide synthesis process is being carried out. Unfortunately, the numerous attempts at this approach have been disappointing. Moore and Beaucage, J. Org. Chem. 50: 2019-2025 (1985) teaches in situ preparation of phosphoramidites by reacting deoxyribonucleosides with bis-(pyrrolidino)methoxyphosphine activated by 4,5-dichloroimidazole in 1-methyl-2-pyrrolidinone. However, this method was limited by poor chemoselectivity, with about 8-10% (3'-3')-dinucleoside methyl phosphite triester being formed as a by-product. Barone *et al*., Nucleic Acids Res. 12: 4051-4061 (1984) and Lee and Moon, Chem. Lett. 1229-1232 (1984) disclose better chemoselectivity in preparation of phosphoramidites *in situ*, by reacting deoxyribonucleosides with bis-(N,N,-dialkylamino)alkoxyphosphines and 1H-tetrazole or its N,N-diisopropylammonium salt. Unfortunately, the tetrazole-N,N-diisopropylammonium salt, either added or generated *in situ* may form precipitates inside the synthesizer. Helinski *et al*., Tetrahedron Lett. 32: 4981-4984 (1991) and 34: 6451 - 6454 (1993) disclose selective activation of bifunctional phosphitylating reagents containing a p-nitrophenoxy group. However, this methodology is not adaptable to current phosphoramidite approaches because the p-nitrophenoxy group has to be activated by using a strong base. Finally, Fourrey *et al*., Tetrahedron Lett. 22: 729-732 (1981) and Cao *et al*., Tetrahedron Lett. 24: 1019-1020 (1983) disclose, as reactive bifunctional phosphitylating agents, phosphorodichlorite and the corresponding ditetrazolite and ditriazolite. Unfortunately, the application of these agents to the synthesis of oligonucleotides is generally problematic, because of their extremely high reactivity and poor chemoselectivity. Further methoxy phosphines are disclosed in WO-A-8 673 62.

There have also been reports of using methylphosphordiamidites to produce nucleoside methylphosphonamidites for oligonucleotide synthesis. Engels *et al*., Nucl. Acids Res. Symposium Series No. 24, pp. 83-86 (1991), discloses the use of methylphosphordiamidites to produce nucleoside methylphosphonamidite monomers for stereoselective synthesis of oligonucleoside methylphosphonates. However, the monomers were purified prior to their use in synthesis, rather than being prepared *in situ*, and sufficient chemoselectivity for the latter approach was not demonstrated.

There is, therefore, a need for new bifunctional phosphitylating reagents and their application in *in situ* preparation of 5,1-protected nucleoside phosphoramidites and P-substituted phosphonamidites and subsequent synthesis of oligonucleotides without prior purification of the nucleoside phosphoramidites or P-substituted phosphonamidites. Ideally, such reagents should be selectively activated and react quickly with nucleosides, should generate chemoselectively the corresponding nucleoside phosphoramidites or P-substituted phosphonamidites *in situ*, and should be relatively stable and easy to handle.

### BRIEF SUMMARY OF THE INVENTION

The invention provides novel bifunctional phosphitylating reagents and novel processes for *in situ* preparation of 5'-protected nucleoside phosphoramidites and P-substituted phosphonamidite monomers and synthesis of oligonucleotides. Bifunctional phosphitylating reagents according to the invention react quickly with nucleosides under weakly acidic conditions. In addition, the bifunctional phosphitylating reagents according to the invention generate chemoselectively the corresponding nucleoside phosphoramidite or P-substituted phosphonamidite monomers *in situ*, without the need to purify the nucleoside phosphoramidite or P-substituted phosphonamidite monomers before using them in oligonucleotide synthesis. Finally, the bifunctional phosphitylating reagents according to the invention are relatively stable and easy to handle.

In a first aspect, the invention provides bifunctional phosphitylating reagents which are useful for *in situ* preparation of 5'-protected nucleoside phosphoramidite or P-substituted phosphonamidite monomers and synthesis of oligonucleotides. For purposes of the invention, a nucleoside P-substituted phosphonamidite is a nucleoside phosphonamidite in which a non-bridging oxygen atom of the corresponding phosphoramidite has been replaced with an organic substituting group. Organic substituting groups have from one to 20 carbon atoms and include alkyl, aryl, aralkyl, alkoxy, aroxy, aralkoxy, thioalkyl, thioaryl, or thioaralkyl groups, any of which may be unsubstituted or up to fully substituted with halogen and or nitrogen constituents. Particularly preferred organic substituting groups include CH₃O-, NCC₂H₄O-, CH₃-, NCC₂H₄S-, or PhCOSCH₂CH₂S- groups, wherein Ph is phenyl or 2,4-dichlorophenyl. Bifunctional phosphitylating reagents according to this aspect of the invention have the general structure (II): 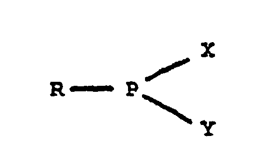 wherein R is an alkyl, aryl, aralkyl, alkoxy, aroxy, aralkoxy, thioalkyl, thioaryl, or thioaralkyl group having from one to 20 carbon atoms and being unsubstituted or up to fully substituted with halogen and or nitrogen constituents, more preferably being CH₃O-, NCC₂H₄O-, CH₃-, NCC₂H₄S-, or PhCOSCH₂CH₂S-, wherein Ph is phenyl or 2,4-dichlorophenyl;
and wherein X and Y are different from each other and are independently selected from the group consisting of 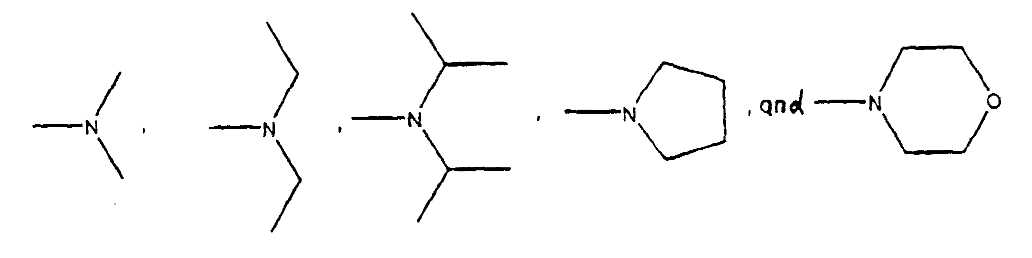 Bifunctional phosphitylating reagents according to this aspect of the invention react in the presence of a weak acid with 5'-protected nucleosides to chemoselectively produce 5'-projected nucleoside phosphoramidite or P-substituted phosphonamidite monomers.

In a second aspect, the invention provides a process for generating 5'-protected nucleoside phosphoramidite or P-substituted phosphonamidite monomers without producing a precipitate and without requiring purification of the nucleoside phosphoramidite or P-substituted phosphonamidite monomers prior to their use in oligonucleotide synthesis. In the process according to this aspect of the invention, bifunctional phosphitylating reagents according to the first aspect of the invention are reacted with 5'-protected nucleosides in the presence of a weak acid to produce 5'-protected nucleoside phosphoramidite or P-substituted phosphonamidite monomers.

In a third aspect, the invention provides an improved process for synthesizing oligonucleotides. In the process according to this aspect of the invention, the improvement comprises the step of generating the nucleoside phosphoramidite or P-substituted phosphonamidite monomers *in situ*, rather than adding purified nucleoside phosphoramidite or P-substituted phosphonamidite monomers at the appropriate point in a conventional oligonucleotide synthesis procedure. The *in situ* generation preferably utilizes the phosphitylating agents according to the first aspect of the invention.

The reagents and processes according to the invention are useful for producing a wide variety of oligonucleotide or P-substituted oligonucleotide compounds, or radiolabeled oligonucleotide or P-substituted compounds, all of which are referred to herein generally as "oligonucleotides". The reagents and processes according to the invention can be used or practiced on a scale ranging from a small laboratory scale to a large commercial scale.

### BRIEF DESCRIPTION OF THE DRAWING

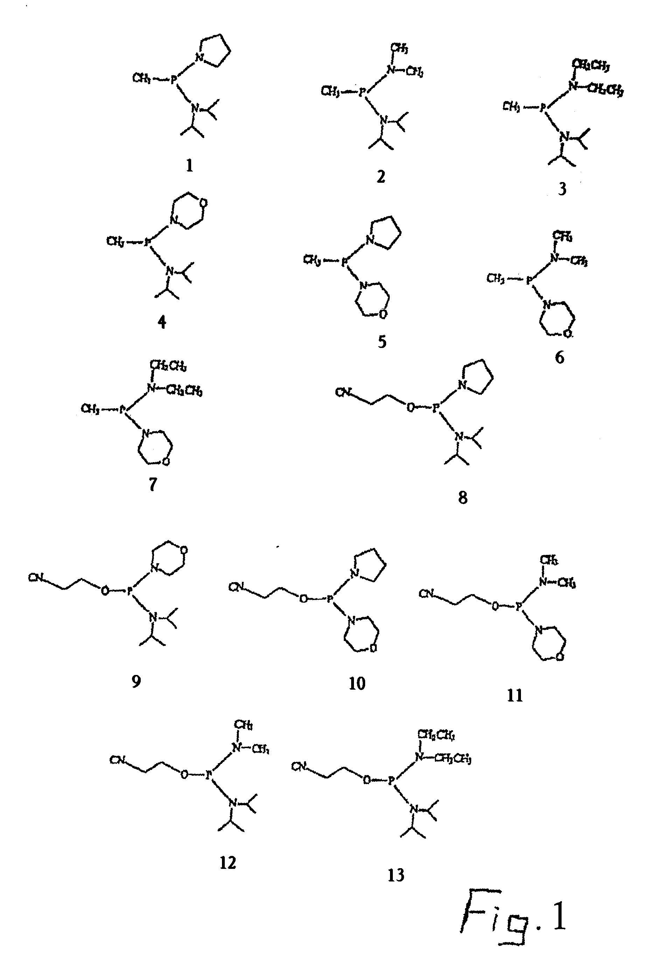

Figure 1 shows 13 particularly preferred embodiments of bifunctional phosphitylating reagents according to the first aspect of the invention.

### DETAILED DESCRIPTION OF THE PREFERRED EMBODIMENTS

The invention relates to the chemical synthesis of oligonucleotides and to chemical entities useful in such synthesis. The patents and publications identified in this specification are within the knowledge of those skilled in this field and are hereby incorporated by reference in their entirety.

The invention provides novel bifunctional phosphitylating reagents and novel processes for *in situ* preparation of 5'-protected nucleoside phosphoramidite or P-substituted phosphonamidite monomers and synthesis of oligonucleotides. Bifunctional phosphitylating reagents according to the invention react quickly with nucleosides under weakly acidic conditions. In addition, the bifunctional phosphitylating reagents according to the invention generate chemoselectively the corresponding nucleoside P-substituted phosphonamidite monomers *in situ*, without the need to purify the nucleoside P-substituted phosphonamidite monomers before using them in oligonucleotide synthesis. Finally, the bifunctional phosphitylating reagents according to the invention are relatively stable and easy to handle.

In a first aspect, the invention provides bifunctional phosphitylating reagents which are useful for *in situ* preparation of 5'-protected nucleoside phosphoramidite or P-substituted phosphonamidite monomers and synthesis of oligonucleotides. For purposes of the invention, a nucleoside P-substituted phosphonamidite is a nucleoside phosphonamidite in which a non-bridging oxygen atom of the corresponding phosphoramidite has been replaced with an organic substituting group. Organic substituting groups have from one to 20 carbon atoms and include alkyl, aryl, aralkyl, alkoxy, aroxy, aralkoxy, thioalkyl, thioaryl, or thioaralkyl groups, any of which may be unsubstituted or up to fully substituted with halogen and or nitrogen constituents. Particularly preferred organic substituting groups include CH₃O-, NCC₂H₄O-, CH₃-, NCC₂H₄S-, or PhCOSCH₂CH₂S- groups, wherein Ph is phenyl or 2,4-dichlorophenyl. Bifunctional phosphitylating reagents according to this aspect of the invention have the general structure (II): 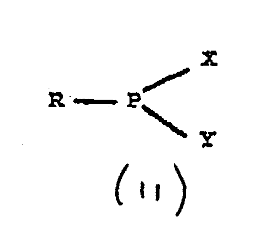 wherein R is an alkyl, aryl, aralkyl, alkoxy, aroxy, aralkoxy, thioalkyl, thioaryl, or thioaralkyl, group having from one to about 20 carbon atoms and being unsubstituted or up to fully substituted with halogen and or nitrogen constituents, more preferably being CH₃O-, NCC₂H₄O-, CH₃-, NCC₂H₄S-, or PhCOSCH₂CH₂S-, wherein Ph is phenyl or 2,4-dichlorophenyl;
and wherein X and Y are different from each other and are independently selected from the group consisting of 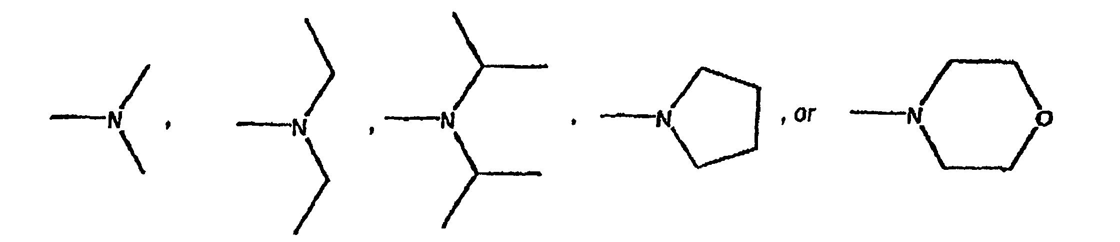 provided that when R is methoxy, X or Y is not diisopropylamino when the other is morpholino.

Bifunctional phosphitylating reagents according to this aspect of the invention can be synthesized as described in Examples 1-13, below, or by simple adaptation of these Examples. Bifunctional phosphitylating reagents according to this aspect of the invention react in the presence of a weak acid with 5'-protected nucleosides to chemoselectively produce 5'-protected nucleoside-P-substituted phosphonamidite monomers. To avoid formation of a nucleoside 3'-3' dimer byproduct, the *in situ* activation using these reagents is preferably carried out using as a weak acid 0.25-0.3 equivalents of tetrazole or 4,5-dichloroimidazole.

In a second aspect, the invention provides processes for generating 5'-protected nucleoside phosphoramidites or P-substituted phosphonamidites, without producing a precipitate and with sufficient chemoselectivity to eliminate the need for purification of the nucleoside phosphoramidites or P-substituted phosphonamidites so formed. In the process according to this aspect of the invention, bifunctional phosphitylating reagents according to the first aspect of the invention are reacted with 5'-protected nucleosides in the presence of a weak acid to produce a 5'-protected nucleoside phosphoramidite or P-substituted phosphonamidite. Preferred 5'-protected nucleosides include adenosine, guanosine, cytosine, uridine, incline and thymidine, as well as modified nucleosides (see *e.g.*, Sanghvi, in Antisense Research and Applications, pp. 273-288 (Crook and Lebleu, Eds.) CRC Press (1993) and the references cited therein). The 5' position of the nucleoside may be protected by any of the standard protecting groups (see *e.g*., Sonveaux in Protocols for Oligonucleotide Conjugates, pp. 1-72 (S. Agrawal, Ed.), Humana Press (1994)) or with any protective group suitable for oligonucleotide synthesis. In certain preferred embodiments, the 5' position of the nucleoside is protected by a dimethoxytrityl (DMT) group.

The reaction between the bifunctional phosphitylation reagent and the 5'-protected nucleoside can be monitored by conventional ³¹P NMR spectroscopy. The most preferred bifunctional phosphitylation reagents according. to the invention will react to completion with the 5'-protected nucleoside within about 10 minutes.

In the process according to this aspect of the invention, to obtain chemoselectivity of the reaction for the desired 5'-protected nucleoside P-substituted phosphonamidite, the concentration and nature of the activator is controlled. Thus, the activation is preferably carried out using as a weak acid about 0.25-0.3 equivalents of tetrazole or 4,5-dichloroimidazole. In this context, "about" means approximately plus or minus 3%. These conditions lead to rapid synthesis of the desired 5'-protected nucleoside P-substituted phosphonamidite, with contamination by the nucleoside 3'-3' dimer at a level of only 3% or less.

In a third aspect, the invention provides process for synthesizing oligonucleotides. In the process according to this aspect of the invention, the improvement comprises the step of generating the nucleoside phosphoramidite or P-substituted phosphonamidite monomers *in situ*, rather than adding purified nucleoside phosphoramidite or P-substituted phosphonamidite monomers at the appropriate point in a conventional oligonucleotide synthesis procedure. The *in situ* generation preferably utilizes the phosphitylating agents according to the first aspect of the invention. Some of the oligonucleotides synthesized according to this aspect of the invention will be P-substituted oligonucleotides. For purposes of the invention, a P-substituted oligonucleotide is an oligonucleotide in which from one to about all of the internucleoside phosphorous atoms has one non-bridging oxygen atom from the corresponding phosphodiester substituted with an organic substituting group. Organic substituting groups have from one to 20 carbon atoms and include alkyl, aryl, aralkyl, alkoxy, aroxy, aralkoxy, thioalkyl, thioaryl, or thioaralkyl groups, any of which may be unsubstituted or up to fully substituted with halogen and or nitrogen constituents. Particularly preferred organic substituting groups include CH₃O-, NCC₂H₄O-, CH₃-, NCC₂H₄S-, or PhCOSCH₂CH₂S- groups, wherein Ph is phenyl or 2,4-dichlorophenyl. In another preferred embodiment, another non-bridging oxygen atom from the corresponding phosphodiester is replaced by a sulfur atom.

For purposes of the invention, the term *in situ* is intended to mean "without intervening purification". Thus, generating 5'-protected nucleoside P-substituted phosphonamidites *in situ* takes place whenever at least one of the 5'-protected nucleoside P-substituted phosphonamidites are generated and then used for oligonucleotide synthesis without intervening purification of the 5'-protected nucleoside P-substituted phosphonamidites. Thus, the improved process for synthesizing P-substituted oligonucleotides according to the invention comprises generating a 5'-protected nucleoside phosphoramidite or P-substituted phosphonamidite *in situ* and coupling the P-substituted phosphonamidite or the 5'-protected nucleoside-P- substituted phosphoramidite with an unprotected 5' end of a nucleoside, which is preferably covalently bound to a solid support and which may be the 5'-terminal nucleoside of a nascent oligonucleotide. The generation of the 5'-protected nucleoside phosphoramidites or P-substituted phosphonamidites and synthesis of oligonucleotides may take place in the same reaction vessel as the nucleoside coupling reactions, or it may take place in different reaction vessels. Moreover, the generation of 5'-protected nucleoside phosphoramidites or P-substituted phosphonamidites may take place either prior to, or contemporaneous with oligonucleotide synthesis.

In a particularly preferred embodiment of the process according to this aspect of the invention, the biphosphitylating agent used to produce the nucleoside phosphoramidites or P-substituted phosphonamidites *in situ* is selected from the biphosphitylating agents shown in Figure 1. The preferred nucleoside P-substituted phosphonamidite is generated *in situ*, followed by coupling without intervening purification of the nucleoside P-substituted phosphonamidite.

The improvement according to this aspect of the invention can be incorporated into any standard phosphoramidite synthesis protocol using any automated synthesizer.

The versatility of the improvement according to this aspect of the invention allows it to be used for the synthesis of a wide variety of different oligonucleotides. For purposes of the invention, the term "oligonucleotide" includes polymers of two or more deoxyribonucleotide or 2'-O-substituted ribonucleotide monomers, or any combination thereof. Such monomers may be coupled to each other by any of the numerous known internucleoside linkages. In certain preferred embodiments, these internucleoside linkages may be phosphodiester, phosphotriester, phosphorothioate, phosphorodithioate, methylphosphonate, or phosphoramidate linkages, or combinations thereof. The term oligonucleotide also encompasses such polymers having chemically modified or radioisotopically labeled bases or sugars and/ or having additional substituents, including without limitation lipophilic groups, intercalating agents, diamines and adamantane. For purposes of the invention the term "2'-O-substituted" means substitution of the 2' position of the pentose moiety with an -O- alkyl group containing 1-6 saturated or unsaturated carbon atoms, or with an -O-aryl or allyl group having 2-6 carbon atoms, wherein such alkyl, aryl or allyl group may be unsubstituted or may be substituted, *e.g*., with halo, hydroxy, trifluoromethyl, cyano, nitro, acyl, acyloxy, alkoxy, carboxyl, carbalkoxyl, or amino groups; or such 2' substitution may be with a hydroxy group (to produce a ribonucleoside), an amino or a halo group, but not with a 2'-H group.

The following examples are intended to further illustrate certain preferred embodiments of the invention and are not intended to be limiting in nature. Except as otherwise indicated, in each of the following examples, reagents were sourced as follows. Anhydrous acetonitrile was purchased from J. T. Baker Inc. (Phillipsburg, NJ). dT-CPG, 5'-DMT-deoxyadenosine (Bz) cyanoethyl phosphoramidite, 5'-DMT-deoxycytidine (Bz) cyanoethyl phosphoramidite, 5'-DMT-deoxyguanosine (ibu) Cyanoethyl phosphoramidite, 5'-DMT-thymidine Cyanoethyl phosphoramidite, Cap A, Cap B, activator, oxidizing and deblock solutions were purchased from PerSeptive Biosystems, (Framingham, MA). Ammonia solution in methanol (ca . 7N) was purchased from ACROS ORGANIC (Pittsburgh, PA). All other chemicals were purchased from Aldrich. ³¹P NMR spectra (121.65 MHz) and ¹H NMR spectra (300 MHz) were recorded on a Varian UNITY 300 (the chemical shift was correlated to 85% H₃PO₄ and tetramethylsilane, respectively). Oligonucleotide synthesis was performed on a 8909 Expedite™ DNA synthesizer (Millipore). Compound numbers, shown in bold, refer to the compounds shown in Figure 1.

### Example 1

### Synthesis of 2-Cyanoethoxy(N,N-Diisopropylamino) pyrrolidinophosphine (1).

To a solution of chloro(2-Cyanoethoxy) (N,N-Diisopropylamino) phosphine (12.9 g, 54.56 mmol) in CH₂Cl₂ (100 mL) was added dropwise 1-(trimethylsilyl)pyrrolidine (10.0 mL, 8.21 g, 57.29 mmol) at room temperature. The resulting Mixture was stirred overnight at room temperature. The solvent was removed under reduced pressure to give a pale yellow oil (13.3 g, 95%) as a product. ³¹P NMR (CDCl₃) δ 133.9.

### Example 2

### Synthesis of 2-Cyanoethoxy(N,N-Diisopropylamino) (N,N-dimethylamino)phosphine (2)

To a solution of chloro(2-Cyanoethoxy) (N,N-Diisopropylamino) phosphine (19,22 g, 81.2 mmol, 18.1 mL) in CH₂Cl₂ (100 mL) was added dropwise N,N-dimethyltrimethylsilylamine (10.0 g, 85.3 mmol) at room temperature. The resulting mixture was stirred overnight at room temperature. The solvent was removed under reduced pressure to give a colorless oil (18.7 g. 94%) as a product. ³¹P NMR (CDCl₃) δ 126.3.

### Example 3

### Synthesis of 2-Cyanoethoxy(N,N-Diethylamino) (N,N-diisopropylamino) phosphine (3).

To solution of chloro(2-Cyanoethoxy) (N,N-Diisopropylamino) phosphine (5.95 g, 25 .13 mmol, 5.61 mL) in CH₂Cl₂ (50 mL) was added dropwise N,N-Diethyltrimethylsilylamine (3.84 g, 26.4 mmol, 5.0 mL) at room temperature. The resulting mixture was stirred overnight at room temperature. The solvent was removed under reduced pressure to give a colorless oil (6.5 g, 94% as a product. ³¹P NMR (CDCl₃) δ 127.2 .

### Example 4

### Synthesis of 2-Cyanoethoxy (N,N-Diisopropylamino) morpholinophosphine (4).

To a solution of chloro (2-Cyranoethoxy) (N,N-Diisopropylamino) phosphine (10.61 g, 44.82 mmol, 10.0 mL) in CH₂Cl₂ (50 mL) was added dropwise 4-(trimethylsilyl)morpholine (8.76 mL, 7.86 g, 49.31 mmol) at room temperature. The resulting mixture was stirred overnight at room temperature. The solvent was removed under reduced pressure to give a colorless oil (12.5 g, 97%) as a product. ³¹P NMR (CDCl₃) δ 125.2.

### Example 5

### Synthesis of 2-Cyanoethoxy(morpholino) pyrrolidinophosphine (5).

To a solution of 2-Cyanoethoxy(dichloro)phosphine (7.16 g, 41.61 mmol, 5.3 mL) in CH₂Cl₂ (50 mL) was added dropwise 1-(trimethylsilyl)pyrrolidine (7.26 mL, 5.96 g, 41.61 mmol) at room temperature. The resulting mixture was stirred overnight at room temperature. The solvent was removed under reduced pressure to give chloro(2-Cyanoethoxy)pyrrolidinophosphine as a colorless oil. ³¹P NMR (CDCl₃) δ 177.5.

To the solution of chloro (2-Cyanoethoxy) pyrrolidinophosphine in CH₂Cl₂ (50 mL) was added dropwise 4-(trimethylsilyl)morpholine (8.13 mL, 7.29 g, 45.77 mmol) at room temperature. The resulting mixture was stirred overnight at room temperature. The solvent was removed under reduced pressure to give a pale yellow oil (9.8 g, 92%) as a product. ³¹P NMR (CDCl₃) δ 134.2

### Example 6

### Synthesis of 2-Cyanoethoxy(N,N-dimethylamino) morpholinophosphine (6).

To a solution of 2-Cyanoethoxy (dichloro)phosphine (4.29 g, 24.97 mmol, 3.2 mL) in CH₂Cl₂ (50 mL) was added dropwise N,N-dimethyltrimethylsilylamine (2.93 g, 24.97 mmol, 4.0 mL) at room temperature. The resulting mixture was stirred overnight at room temperature. The solvent was removed under reduced pressure to give chloro(2-Cyanoethoxy) (N,N-dimethylamino)phosphine as a colorless oil. ³¹P NMR (CDCl₃) δ 174.7.

To the solution of chloro (2-Cyanoethoxy) (N,N-dimethylamino)phosphine in CH₂Cl₂ (50 mL) was added dropwise 4-(trimethylsilyl) morpholine (4.86 mL, 4.36 g, 27.39 mmol) at room temperature. The resulting mixture was stirred overnight at room temperature. The solvent was removed under reduced pressure to give a pale yellow oil (5.0 g, 93%) as a product. ³¹P NMR (CDCl₃) δ 133.5.

### Example 7

### Synthesis of N,N-Diisopropylamino(methyl) pyrrolidinophosphine (7).

To a solution of chloro(N,N-Diisopropylamino)methylphosphine (5.0 g, 27.53 mmol) in CH₂Cl₂ (50 mL) was added dropwise 1-(trimethylsilyl)pyrrolidine (5.3 mL) 4.34 g, 30.3 mmol) at room temperature. The resulting mixture was stirred overnight at room temperature. The solvent was removed under reduced pressure to give a colorless oil (5.5 g. 93%) as a product. ³¹P NMR (CDCl₃) δ 48.7.

### Example 8

### Synthesis of N,N-Diisopropylamino(methyl) (N,N-dimethylamino)phosphine (8).

To a solution of chloro(N,N-Diisopropylamino)methylphosphine (6.2 g, 34.1 mmol, 6.2 mL) in CH₂Cl₂ (50 mL) was added dropwise N,N-dimethyltrimethylsilylamine (4.39 g, 37.45 mmol) at room temperature. The resulting mixture was stirred overnight at room temperature. The solvent was removed under reduced pressure to give a colorless oil (4.3 g, 90%) as a product. ³¹P NMR (CDCl₃) δ 50.6.

### Example 9

### Synthesis of N,N-Diethylamino (N,N-diisopropylamino)methylphosphine (9).

To a solution of chloro(N,N-Diisopropylamino)methylphosphine (5.0 g, 27.53 mmol) in CH₂Cl₂ (50 mL) was added dropwise N,N-diethyltrimethylsilylamine (4.4 g, 30.28 mmol, 5.7 mL) at room temperature. The resulting mixture was stirred overnight at room temperature. The solvent was removed under reduced pressure to give a colorless oil (5.0 g, 83%) as a product . ³¹P NMR (CDCl₃) δ 56.6.

### Example 10

### Synthesis of N,N-Diisopropylamino (methyl)morpholinophosphine (10).

To a solution of chloro(N,N-Diisopropylamino)methylphosphine (5.0 g, 27.53 mmol, 5.0 mL) in CH₂Cl₂ (50 mL) was added dropwise 4-(trimethylsilyl)morpholine (5.4 mL, 4.8 g, 30.28 mmol) at room temperature. The resulting mixture was stirred overnight at room temperature. The solvent was removed under reduced pressure to give a pale yellow oil (6.1 g, 96%) as a product. ³¹P NMR (CDCl₃) δ 58.8.

### Example 11

### Synthesis of Methyl (morpholino)pyrrolidinophosphine (11).

To a solution of mathyldichlorophosphine (5.0 g, 42.76 mmol) in CH₂Cl₂ (50 mL) was added dropwise 1-(trimethylsilyl)pyrrolidine (7.5 mL, 6.1 g, 42.8 mmol) at room temperature. The resulting mixture was stirred overnight at room temperature. The solvent was removed under reduced pressure to give chloro(methyl) pyrrolidinophosphine as a colorless oil. ³¹P NMR (CDCl₃) δ 144.8.

To the solution of chloro(methyl)pyrrolidinophosphine in CH₂Cl₂ (50 mL) was added dropwise 4-(trimethylsilyl)morpholine (8.3 mL, 7.5 g, 43.0 mmol) at room temperature. The resulting mixture was stirred overnight at room temperature. The solvent was removed under reduced pressure to give a pale yellow oil (8.2 g, 95%) as a product. ³¹P NMR (CDCl₃) δ 72.5.

### Example 12

### Synthesis of Methyl(N,N-dimethylamino) morpholinophosphine (12).

To a solution of methyldichlorophosphine (5.0 g, 42.76 mmol, 3.8 mL) in CH₂Cl₂ (50 mL) was added dropwise N,N-dimethyl-trimethylsilylamine (5.0 g, 42.76 mmol, 6.9 mL) at room temperature. The resulting mixture was stirred overnight at room temperature. The solvent was removed under reduced pressure to give chloro (methyl) (N,N-dimethylamino)phosphine as a colorless oil. ³¹P NMR (CDCl₃) δ 151.3.

To the solution of chloro (methyl) (N,N-dimethylamino) phosphine in CH₂Cl₂ (50 mL) was added dropwise 4-(trimethylsilyl)morpholine (8.3 mL, 7.5 g, 43.0 mmol) at room temperature. The resulting mixture was stirred overnight at room temperature. The solvent was removed under reduced pressure to give a pale yellow oil (7.1 g, 94%) as a product. ³¹P NMR (CDCl₃) δ 81.6.

### Example 13

### Synthesis of N,N-Diethylamino (methyl)morpholinophosphine (13).

To a solution of methyldichlorophosphine (10 g, 85.5 mmol, mL) in CH₂Cl₂ (50 mL) was added dropwise N,N-diethyltrimethylsilylamine (12.43 g, 16.2 mmol) at room temperature. The resulting mixture was stirred overnight at room temperature. The solvent was removed under reduced pressure to give chloro(methyl) (N,N-dimethylamino)phosphine as a colorless oil. ³¹P NMR (CDCl₃) δ 147.8.

To the solution of chloro(methyl) (N,N-dimethylamino) phosphine (11.4 g, 81. 77 mmol) in CH₂Cl₂ (50 mL) was added dropwise 4-(trimethylsilyl)morpholine (17.43 mL, 15.63 g, 98.12 mmol) at room temperature. The resulting mixture was stirred overnight at room temperature. The solvent was removed under reduced pressure to give a pale yellow oil (14.8 g, 89%) as a product. ³¹P NMR (CDCl₃) δ 81.4.

## Claims

1. A bifunctional phosphitylating reagent having the general structure (II): 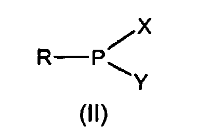 wherein R is an alkyl, aryl, aralkyl, alkoxy, aroxy, aralkoxy, thioalkyl, thioaryl, or thioaralkyl group having from one to 20 carbon atoms and being unsubstituted or up to fully substituted with halogen and or nitrogen constituents;
and wherein X and Y are different from each other and are independently selected from the group consisting of 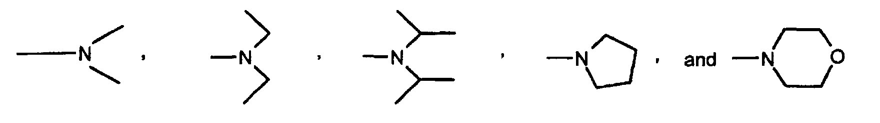 provided that when R is methoxy, X or Y is not diisopropylamino when the other is morpholino.

2. The bifunctional phosphitylating reagent according to claim 1 wherein R is CH₃O-, NCC₂H₄O-, CH₃-, NCC₂H₄S-, or PhCOSCH₂S-, wherein Ph is phenyl or 2,4-dichlorophenyl.

3. A process for generating 5'-protected nucleoside phosphoramidites or P-substituted phosphonamidites, the process comprising reacting a bifunctional phosphitylating reagent having the general structure (II): 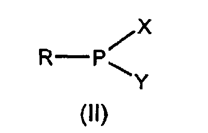 wherein R is an alkyl, aryl, aralkyl, alkoxy, aroxy, aralkoxy, thioalkyl, thioaryl, or thioaralkyl group having from one to 20 carbon atoms and being unsubstituted or up to fully substituted with halogen and or nitrogen constituents;
and wherein X and Y are different from each other and are independently selected from the group consisting of 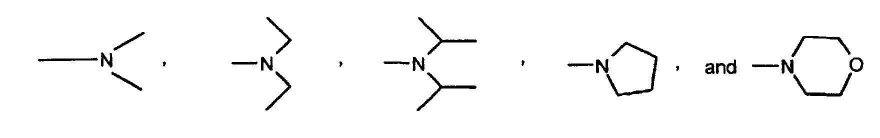 provided that when R is methoxy, X or Y is not diisopropylamino when the other is morpholino;
with a 5'-protected nucleoside in the presence of a weak acid to produce a 5'-protected nucleoside phosphoramidite or P-substituted phosphonamidite.

4. The process according to claim 3, wherein R is CH₃O-, NCC₂H₄O-, CH₃-, NCC₂H₄S-, or PhCOSCH₂CH₂S-, wherein Ph is phenyl or 2,4-dichlorophenyl.

5. A process for synthesising an oligonucleoside containing one or more P-substituted internucleoside linkage, the improvement comprising generating a 5'-protected nucleoside phosphoramidite or nucleoside P-substituted phosphonamidite *in situ* by reacting a bifunctional phosphitylating reagent having the general structure (II): 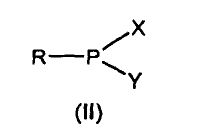 wherein R is an alkyl, aryl, aralkyl, alkoxy, aroxy, aralkoxy, thioalkyl, thioaryl, or thioaralkyl group having from one to 20 carbon atoms and being unsubstituted or up to fully substituted with halogen and or nitrogen constituents;
and wherein X and Y are different from each other and are independently selected from the group consisting of 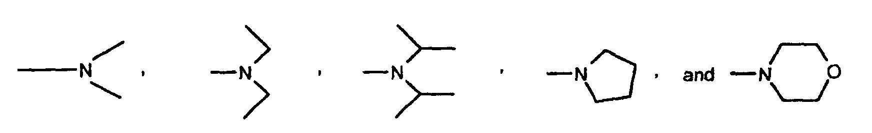 with a 5'-protected nucleoside in the presence of a weak acid to produce a 5'-protected nucleoside phosphoramidite or 5'-protected nucleoside P-substituted phosphonamidite.

6. The process according to claim 5, wherein R is CH₃O-, NCC₂H₄O-, CH₃-, NCC₂H₄S-, or PhCOSCH₂CH₂S-, wherein Ph is phenyl or 2,4-dichlorophenyl.

7. A process according to claim 5 for synthesising P-substituted oligonucleotides, comprising generating a 5'-protected nucleoside-P-substituted phosphonamidite *in situ* and coupling the P-substituted phosphonamidite of the 5'-protected nucleoside-P-substituted phosphonamidite with an unprotected 5' end of a nucleoside, **characterised in that** the 5'-protected nucleoside-P-substituted phosphonamidite is prepared from a compound according to claim 1 wherein R is an alkyl, aryl or aralkyl group having from one to 20 carbon atoms and being unsubstituted or up to fully substituted with halogen and or nitrogen constituents.

8. Use of a compound of claim 1 or 2 for the synthesis of oligonucleotides.

## Patentansprüche

1. Bifunktionelles, phosphitylierendes Reagens mit der allgemeinen Struktur (II): 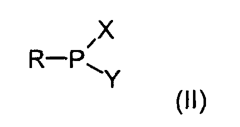 wobei R eine Alkyl-, Aryl-, Aralkyl-, Alkoxy-, Aroxy-, Aralkoxy-, Thioalkyl-, Thioaryl- oder Thioaralkylgruppe mit zwischen einem und 20 Kohlenstoffatomen und entweder nicht substituiert oder mit Halogen- und/oder Stickstoffbestandteilen bis zu vollständig substituiert ist;
und wobei X und Y sich voneinander unterscheiden und unabhängig voneinander aus der Gruppe bestehend aus 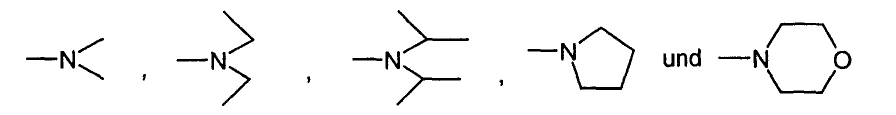 ausgewählt sind, vorausgesetzt, dass, wenn R eine Methoxygruppe ist, es sich bei X bzw. Y nicht um eine Diisopropylaminogruppe handelt, wenn der andere Rest eine Morpholingruppe ist.

2. Bifunktionelles, phosphitylierendes Reagens nach Anspruch 1, wobei R CH₃O-, NCC₂H₄O-, CH₃-, NCC₂H₄S- oder PhCOSCH₂S- ist, worin Ph Phenyl oder 2,4-Dichlorphenyl ist.

3. Verfahren zum Herstellen 5'-geschützter Nukleosidphosphoramidite oder P-substituierter Phosphonamidite, wobei das Verfahren das Umsetzen eines bifunktionellen, phosphitylierenden Reagens mit der allgemeinen Struktur (II) 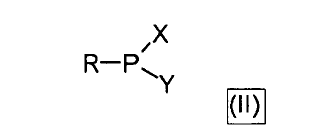 umfasst,
wobei R eine Alkyl-, Aryl-, Aralkyl-, Alkoxy-, Aroxy-, Aralkoxy-, Thioalkyl-, Thioaryl- oder Thioaralkylgruppe mit zwischen einem und 20 Kohlenstoffatomen und entweder nicht substituiert oder mit Halogen- und/oder Stickstoffbestandteilen bis zu vollständig substituiert ist;
und wobei X und Y sich voneinander unterscheiden und unabhängig voneinander aus der Gruppe bestehend aus 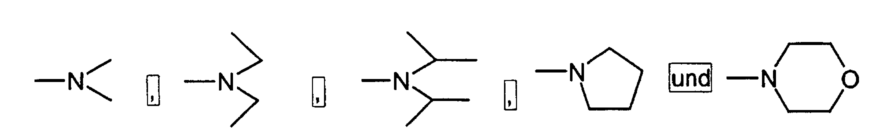 ausgewählt sind, vorausgesetzt, dass, wenn R eine Methoxygruppe ist, es sich bei X bzw. Y nicht um eine Diisopropylaminogruppe handelt, wenn der andere Rest eine Morpholingruppe ist;
mit einem 5'-geschützten Nukleosid in Gegenwart einer schwachen Säure, um ein 5'-geschütztes Nukleosidphosphoramidit oder P-substituiertes Phosphonamidit herzustellen.

4. Verfahren nach Anspruch 3, worin R CH₃O-, NCC₂H₄O-, CH₃-, NCC₂H₄S- oder PhCOSCH₂CH₂S- ist, worin Ph Phenyl oder 2,4-Dichlorphenyl ist.

5. Verfahren zum Synthetisieren eines Oligonukleosids, das eine oder mehrere P-substituierte Internukleosidbindungen enthält, wobei die Verbesserung das Herstellen eines 5'-geschützten Nukleosidphosphoramidits oder Nukleosid-P-substituierten Phosphonamidits in situ durch Umsetzen eines bifunktionellen, phosphitylierenden Reagens mit der allgemeinen Struktur (II) 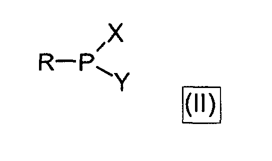 umfasst,
wobei R eine Alkyl-, Aryl-, Aralkyl-, Alkoxy-, Aroxy-, Aralkoxy-, Thioalkyl-, Thioaryl- oder Thioaralkylgruppe mit zwischen einem und 20 Kohlenstoffatomen und entweder nicht substituiert oder mit Halogen- und/oder Stickstoffbestandteilen bis zu vollständig substituiert ist;
und wobei X und Y sich voneinander unterscheiden und unabhängig voneinander aus der Gruppe bestehend aus 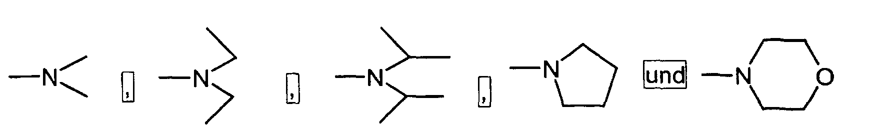 ausgewählt sind;
mit einem 5'-geschützten Nukleosid in Gegenwart einer schwachen Säure, um ein 5'-geschütztes Nukleosidphosphoramidit oder ein 5'-geschütztes Nukleosid-P-substituiertes Phosphonamidit herzustellen.

6. Verfahren nach Anspruch 5, worin R CH₃O-, NCC₂H₄O-, CH₃-, NCC₂H₄S- oder PhCOSCH₂CH₂S- ist, worin Ph Phenyl oder 2,4-Dichlorphenyl ist.

7. Verfahren nach Anspruch 5 zum Synthetisieren von P-substituierten Oligonukleotiden, umfassend das Herstellen eines 5'-geschützten Nukleosid-P-substituierten Phosphonamidits in situ und Koppeln des P-substituierten Phosphonamidits des 5'-geschützten Nukleosid-P-substituierten Phosphonamidits mit einem ungeschützten 5'-Ende eines Nukleosids, **dadurch gekennzeichnet, dass** das 5'geschützte Nukleosid-P-substituierte Phosphonamidit aus einer Verbindung nach Anspruch 1 hergestellt ist, worin R eine Alkyl-, Aryl- oder Aralkylgruppe mit zwischen einem und 20 Kohlenstoffatomen und entweder nicht substituiert oder mit Halogen- und/oder Stickstoffbestandteilen bis zu vollständig substituiert ist.

8. Verwendung einer Verbindung nach Anspruch 1 oder 2 für die Synthese von Oligonukleotiden.

## Revendications

1. Réactif bifonctionnel de phosphitylation de structure générale (II) : 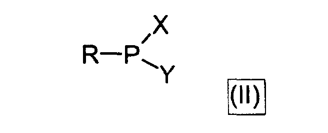 dans laquelle R est un groupe alkyle, aryle, aralkyle, alcoxy, aroxy, aralcoxy, thioalkyle, thioaryle ou thioaralkyle ayant de un à 20 atomes de carbone, et étant non substitué ou jusqu'à totalement substitué par des constituants halogénés et/ou azotés ;
et dans laquelle X et Y sont différents l'un de l'autre et sont choisis indépendamment parmi le groupe constitué de 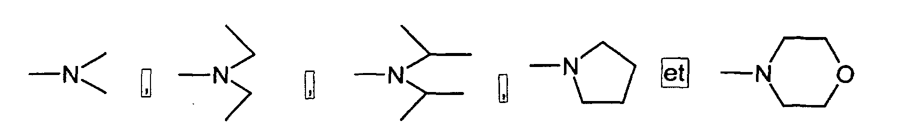 à condition que lorsque R est un méthoxy, X ou Y ne soient pas un diisopropylamino lorsque l'autre est un morpholino.

2. Réactif bifonctionnel de phosphitylation selon la revendication 1, dans lequel R est CH₃O-, NCC₂H₄O-, CH₃-, NCC₂H₄S- ou PhCOSCH₂S-, dans lesquels Ph est un phényle ou un 2,4-dichlorophényle.

3. Procédé de génération de phosphoramidites ou de phosphonamidites P-substitués de nucléosides 5'-protégés, le procédé comprenant la réaction d'un réactif bifonctionnel de phosphitylation de structure générale (II) : 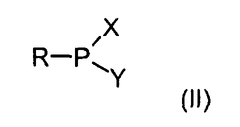 dans laquelle R est un groupe alkyle, aryle, aralkyle, alcoxy, aroxy, aralcoxy, thioalkyle, thioaryle ou thioaralkyle ayant de un à 20 atomes de carbone, et étant non substitué ou jusqu'à totalement substitué par des constituants halogénés et/ou azotés ;
et dans laquelle X et Y sont différents l'un de l'autre et sont choisis indépendamment parmi le groupe constitué de 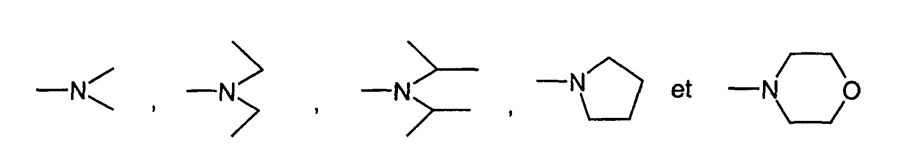 à condition que lorsque R est un méthoxy, X ou Y ne soient pas un diisopropylamino lorsque l'autre est un morpholino ;
avec un nucléoside 5'-protégé en présence d'un acide faible en vue de produire un phosphoramidite ou un phosphonamidite P-substitué de nucléoside 5'-protégé.

4. Procédé selon la revendication 3, dans lequel R est CH₃O-, NCC₂H₄O-, CH₃-, NCC₂H₄S- ou PhCOSCH₂CH₂S-, dans lesquels Ph est un phényle ou un 2,4-dichlorophényle.

5. Procédé de synthèse d'un oligonucléoside renfermant une ou plusieurs liaisons internucléosides P-substituées, l'amélioration comprenant la génération d'un phosphoramidite de nucléoside ou d'un phosphonamidite P-substitué de nucléoside 5'-protégé in situ par réaction d'un réactif bifonctionnel de phosphitylation de structure générale (II) : 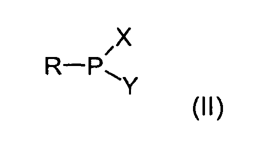 dans laquelle R est un groupe alkyle, aryle, aralkyle, alcoxy, aroxy, aralcoxy, thioalkyle, thioaryle ou thioaralkyle ayant de un à 20 atomes de carbone, et étant non substitué ou jusqu'à totalement substitué par des constituants halogénés et/ou azotés ;
et dans laquelle X et Y sont différents l'un de l'autre et sont choisis indépendamment parmi le groupe constitué de 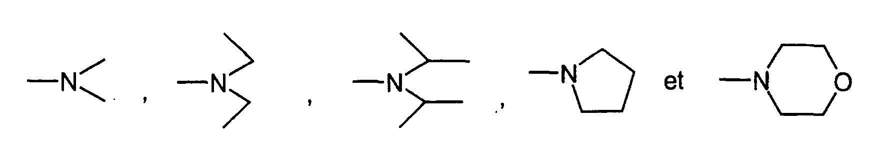 avec un nucléoside 5'-protégé en présence d'un acide faible en vue de produire un phosphoramidite de nucléoside 5'-protégé ou un phosphonamidite P-substitué de nucléoside 5'-protégé.

6. Procédé selon la revendication 5, dans lequel R est CH₃O-, NCC₂H₄O-, CH₃-, NCC₂H₄S- ou PhCOSCH₂CH₂S-, dans lesquels Ph est un phényle ou un 2,4-dichlorophényle.

7. Procédé selon la revendication 5 pour la synthèse d'oligonucléotides P-substitués, comprenant la génération d'un phosphonamidite P-substitué de nucléoside 5'-protégé *in situ* et le couplage du phosphonamidite P-substitué du phosphonamidite P-substitué de nucléoside 5'-protégé avec une extrémité 5' non protégée d'un nucléoside, **caractérisé en ce que** le phosphonamidite P-substitué de nucléoside 5'-protégé est préparé à partir d'un composé selon la revendication 1, dans lequel R est un groupe alkyle, aryle ou aralkyle ayant de un à 20 atomes de carbone, et étant non substitué ou jusqu'à totalement substitué par des constituants halogénés et/ou azotés.

8. Utilisation d'un composé selon la revendication 1 ou 2 pour la synthèse d'oligonucléotides.
